(19) Europäisches Patentamt
European Patent Office
Office européen des brevets

(11) **EP 3 545 777 B1**

(12) **EUROPEAN PATENT SPECIFICATION**

(45) Date of publication and mention
of the grant of the patent:
**06.10.2021 Bulletin 2021/40**

(21) Application number: **16925539.5**

(22) Date of filing: **27.12.2016**

(51) Int Cl.:
***A24F 40/44*** *(2020.01)*

(86) International application number:
**PCT/JP2016/088944**

(87) International publication number:
**WO 2018/122978 (05.07.2018 Gazette 2018/27)**

(54) **HEATING-TYPE FLAVOR INHALER**

GESCHMACKSINHALATOR MIT HEIZFUNKTION

INHALATEUR D'ARÔME DU TYPE À CHAUFFAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**02.10.2019 Bulletin 2019/40**

(73) Proprietor: **Japan Tobacco Inc.**
**Tokyo 105-8422 (JP)**

(72) Inventors:
• **KOMINAMI, Takashi**
  **Tokyo 130-8603 (JP)**

• **TAGUCHI, Hidenari**
  **Tokyo 130-8603 (JP)**

(74) Representative: **Hoffmann Eitle**
**Patent- und Rechtsanwälte PartmbB**
**Arabellastraße 30**
**81925 München (DE)**

(56) References cited:
**WO-A1-2016/147396     JP-A- 2015 513 393**
**JP-A- 2016 539 661     KR-U- 20140 001 494**
**US-A1- 2010 163 062     US-A1- 2014 157 583**

**Description**

FIELD

The present invention relates to an atomization unit and a heating-type flavor inhaler.

BACKGROUND

A heating-type flavor inhaler that atomizes a liquid aerosol source containing a flavor source by heating is known (WO 2013/116558 A1). The heating-type flavor inhaler, unlike a combustion-type smoking article such as a cigarette, does not burn a tobacco material, and thus has the advantage of not emitting smoke generated as an accompaniment to combustion.

US 2014/157583 A1 is related to an apparatus for preforming atomizers for smoking articles, with which the heating element is wound about a wick.

SUMMARY

TECHNICAL PROBLEM

The heating-type flavor inhaler does not burn the tobacco material, and thus has the problem in providing sufficient tobacco-like flavor as compared to the combustion-type smoking article. An object of the present invention is to provide a heating-type flavor inhaler capable of providing
sufficient tobacco-like flavor to a user.

SOLUTION TO PROBLEM

According to one embodiment, there is provided an atomization unit in accordance with claim 1 and a heating-type flavor inhaler in accordance with claim 15.

ADVANTAGEOUS EFFECTS OF INVENTION

According to a heating-type flavor inhaler of one embodiment, it is possible to heat an aerosol source in an atomization chamber containing a flavor source including a tobacco material to thereby atomize the aerosol source and to release a flavor component from the flavor source. In this manner, sufficient tobacco-like flavor can be provided to a user.

BRIEF DESCRIPTION OF THE DRAWINGS

FIG. 1 is a view showing a heating-type flavor inhaler according to an embodiment;
FIG. 2 is a cross-sectional view showing an electrical unit of the heating-type flavor inhaler shown in FIG. 1; and
FIG. 3 is a cross-sectional view showing an atomization unit of the heating-type flavor inhaler shown in FIG. 1.

DETAILED DESCRIPTION

A heating-type flavor inhaler according to the embodiment includes:

an atomization chamber that has a gas inlet port and a gas outlet port, and contains a flavor source including a tobacco material;
a liquid container that contains a liquid aerosol source, and supplies the aerosol source to the flavor source; and
a heater that heats the flavor source supplied with the aerosol source to atomize the aerosol source and to release a flavor component from the flavor source.

A heating-type flavor inhaler according to a preferred embodiment includes:

an outer tubular body that extends in one direction;
an inner tubular body that is positioned in the outer tubular body to be spaced apart from the outer tubular body, and includes an atomization chamber in an inner space;

2

a liquid aerosol source that is contained in a liquid container formed between the outer tubular body and the inner tubular body;

a flavor source that includes a tobacco material, and is disposed such that the flavor source penetrates a pair of through holes provided in the inner tubular body, such that both ends thereof are located in the liquid container to absorb the aerosol source, and such that a center portion thereof is located in the atomization chamber; and

a heater that heats the flavor source, in which the aerosol source is absorbed, to atomize the aerosol source and to release a flavor component from the flavor source. According to a more preferred embodiment, the pair of through holes provided in the inner tubular body is provided on the non-mouthpiece side of the inner tubular body.

According to another embodiment, there is provided an atomization unit that is used in a heating-type flavor inhaler provided with a power source, and atomizes a liquid aerosol source by power supplied from the power source, wherein the atomization unit includes:

an atomization chamber that has a gas inlet port and a gas outlet port, and contains a flavor source including a tobacco material;

a liquid container that contains the liquid aerosol source, and supplies the aerosol source to the flavor source; and

a heater that heats, by the power, the flavor source supplied with the aerosol source to atomize the aerosol source and to release a flavor component from the flavor source.

According to a preferred embodiment, in any of the above-described embodiments, the tobacco material is leaf tobacco. According to a further preferred embodiment, in any of the above-described embodiments, the flavor source including the leaf tobacco is contained in a heat-resistant mesh bag or a heat-resistant mesh container.

According to another preferred embodiment, in any of the above-described embodiments, the tobacco material is a molded body made from a raw material including leaf tobacco. According to a further preferred embodiment, in any of the above-described embodiments, the molded body is a molded body obtained by forming a raw material including leaf tobacco by rolling or papermaking.

"The leaf tobacco" used herein refers to a tobacco material which is ready to be incorporated into a heating-type flavor inhaler through various processing including a drying process of harvested tobacco leaves in a farm house, thereafter an aging process for 1 to several years in a leaf processing plant, and thereafter blending and cutting processes in a manufacturing plant.

According to a preferred embodiment, in any of the above-described embodiments, the molded body is a molded body made from leaf tobacco. According to another preferred embodiment, in any of the above-described embodiments, the molded body is a molded body made from a mixture of leaf tobacco and an absorbent material. According to a further preferred embodiment, in any of the above-described embodiments, the absorbent material is cotton. According to a further preferred embodiment, in any of the above-described embodiments, the mass ratio of the leaf tobacco to the absorbent material is 1:3 to 3:1.

According to a preferred embodiment, in any of the above-described embodiments, the molded body is a bundle of fibrous molded bodies. The "bundle of fibrous molded bodies" can be obtained by cutting a sheet-like molded body into a fibrous form and then bundling the obtained fibrous molded bodies. According to another preferred embodiment, in any of the above-described embodiments, the molded body is a sheet-like molded body, preferably a laminate of sheet-like molded bodies, a sheet-like molded body wound in a spiral shape, or a sheet-like molded body folded in a bellows shape.

According to a preferred embodiment, in any of the above-described embodiments, the liquid container supplies the aerosol source to the flavor source by immersing a part of the flavor source in the aerosol source.

According to a preferred embodiment, in any of the above-described embodiments, the heater is a resistive heating element, preferably a heating wire, wound around the flavor source.

According to a preferred embodiment, in any of the above-described embodiments, the flavor source contains salt. According to a further preferred embodiment, in any of the above-described embodiments, the salt is potassium carbonate, sodium hydroxide or calcium oxide. According to a further preferred embodiment, in any of the above-described embodiments, the flavor source contains the salt in an amount of 5% to 10% by mass, based on the tobacco material.

According to a preferred embodiment, in any of the above-described embodiments, the aerosol source is water, glycerin, propylene glycol or a mixture thereof.

According to a preferred embodiment, in any of the above-described embodiments, the liquid container includes only the aerosol source. That is, the liquid container does not include an additional flavor source.

According to another preferred embodiment, in any of the above-described embodiments, the liquid container includes an additional flavor source. According to a preferred embodiment, the additional flavor source is a flavor source including a tobacco material. According to a more preferred embodiment, the flavor source including the tobacco material is a flavor source including leaf tobacco, a flavor source including a molded body made from a raw material including leaf tobacco, or a flavor source including an extract of leaf tobacco. According to a further preferred embodiment, the flavor source including the tobacco material is a flavor source including a molded body made from a raw material including leaf tobacco. According to another preferred embodiment, the additional flavor source is a flavor source other than the tobacco material. According to a more preferred embodiment, the flavor source other than the tobacco material is a flavor component contained in a liquid of an existing liquid-type electronic cigarette, for example, a flavorant such as menthol.

According to a preferred embodiment, in any of the above-described embodiments, the liquid container further includes a reservoir that holds the aerosol source.

According to a preferred embodiment, in any of the above-described embodiments, the heating-type flavor inhaler further includes a power source that supplies power to the heater. According to a preferred embodiment, in any of the above-described embodiments, the heating-type flavor inhaler further includes a control circuit that controls power supplied to the heater. According to a preferred embodiment, in any of the above-described embodiments, the heating-type flavor inhaler further includes a sensor that outputs, to the control circuit, a response value that varies according to a user's puff action.

[Description of Embodiment With Reference To Drawings]

Hereinafter, a heating-type flavor inhaler according to a specific embodiment will be described with reference to the drawings.

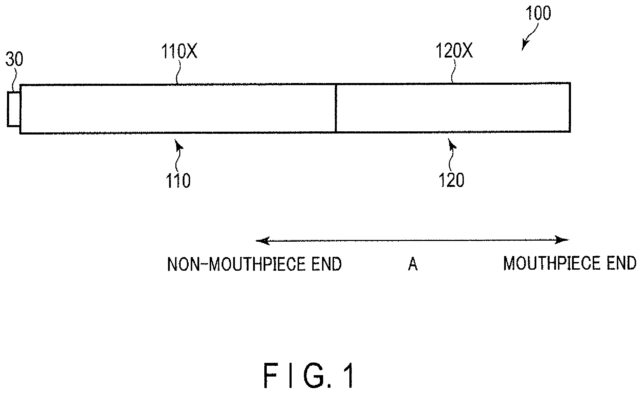

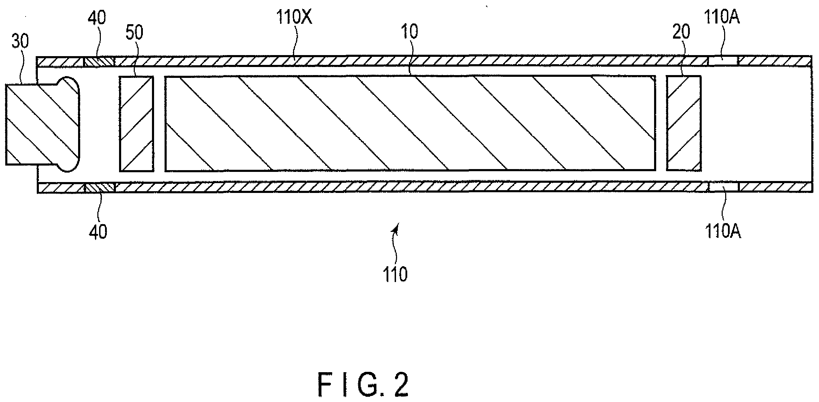

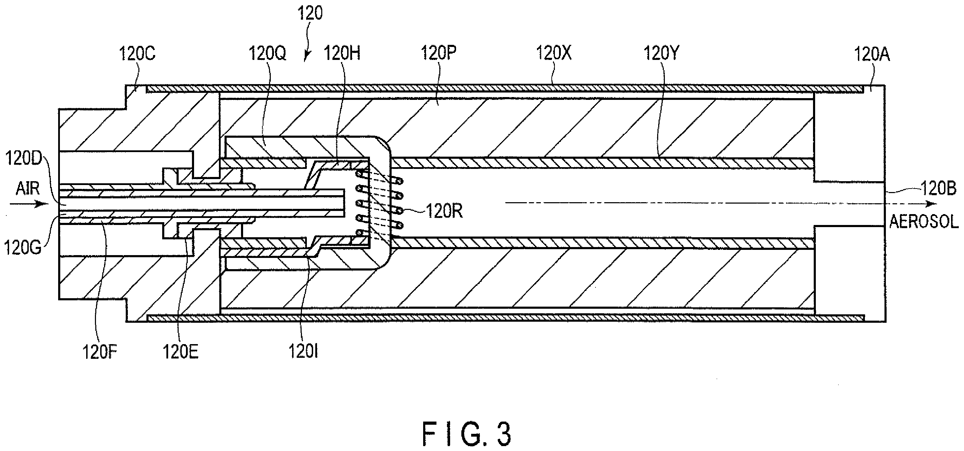

FIG. 1 is a plan view showing a heating-type flavor inhaler according to the embodiment. FIG. 2 is a cross-sectional view showing an electrical unit of the heating-type flavor inhaler shown in FIG. 1. FIG. 3 is a cross-sectional view showing an atomization unit of the heating-type flavor inhaler shown in FIG. 1.

A heating-type flavor inhaler 100 shown in FIG. 1 has a shape extending along direction A, and includes a non-mouthpiece end and a mouthpiece end. The heating-type flavor inhaler 100 may have any shape, and may be, for example, a cylindrical body or a polygonal tubular body.

In the following description, when a "non-mouthpiece side" is referred to for a certain component, a position specified by this "non-mouthpiece side" is an end position of the component, closer to the non-mouthpiece end of the heating-type flavor inhaler 100. In addition, in the following description, when a "mouthpiece side" is referred to for a certain component, a position specified by this "mouthpiece side" is an end position of the component, closer to the mouthpiece end of heating-type flavor inhaler 100.

The heating-type flavor inhaler 100 is a device for inhaling a flavor component by electric heating. In the following description, the heating-type flavor inhaler 100 is simply referred to as a flavor inhaler 100.

The flavor inhaler 100 includes an electrical unit 110 and an atomization unit 120.

As shown in FIG. 2, the electrical unit 110 includes a first tubular body 110X, a power source 10, a sensor 20, a push button switch 30, a light-emitting element 40, and a control circuit 50.

Walls of the first tubular body 110X are provided with gas inlet ports 110A. Ambient air is taken through these gas inlet ports 110A into the first tubular body 110X during inhalation. The air taken into the first tubular body 110X is guided to the atomization unit 120 shown in FIGS. 1 and 3.

The power source 10 is provided inside the first tubular body 110X and between the non-mouthpiece end and the position where the gas inlet ports 110A are provided. The power source 10 is, for example, a lithium-ion battery. The power source 10 supplies power required for operation of the flavor inhaler 100 to electric and electronic components included in the flavor inhaler 100. For example, the power source 10 supplies power to the sensor 20, the light-emitting element 40, and the control circuit 50. Moreover, the power source 10 supplies power to the atomization unit 120 (heater 120R).

The sensor 20 outputs a response value that varies according to the air flow from the non-mouthpiece end toward the mouthpiece end accompanying the inhalation (that is, the user's puff action). The sensor 20 is, for example, a microphone-type pressure sensor.

The push button switch 30 is provided at the non-mouthpiece end of the first tubular body 110X. The push button switch 30 is configured to supply power to the flavor inhaler 100 by being pushed from the outside to the inside of the flavor inhaler 100. For example, when the push button switch 30 is pushed a predetermined number of times within a predetermined time period, the flavor inhaler 100 is powered on. Furthermore, the power of the flavor inhaler 100 is disconnected, for example, when a predetermined time has elapsed since the puff action is performed.

The light-emitting element 40 is a light source such as a light-emitting diode (LED), for example. The light-emitting element 40 is provided on the wall of the first tubular body 110X. The light-emitting element 40 notifies the user of the state of the flavor inhaler 100 by its light emission under the control of the control circuit 50. For example, the light-emitting element 40 emits light in a specific color when power is supplied to the flavor inhaler 100 but no puff action is performed, whereas during puff action, it emits light in a visually distinguished color so as to be differentiated from the aforementioned specific color.

It is preferable that the light-emitting element 40 is provided on the wall of the first tubular body 110X and in the vicinity of the non-mouthpiece end. In this case, the user can easily visually recognize the light emission of the light-emitting element 40 during a puff action, as compared to when the light-emitting element 40 is provided on the central axis of the first tubular body 110X and in the vicinity of the non-mouthpiece end (for example, in the push button switch 30).

The control circuit 50 controls the operation of the flavor inhaler 100. Specifically, the control circuit 50 controls the light emission operation of the light-emitting element 40 and the power to be supplied to the atomization unit 120 (heater 120R), based on the response value output from the sensor 20. For example, the control circuit 50 causes the light-emitting element 40 to emit light only during the period when the power is turned on, and causes the light emitting state of the light-emitting element 40 to be different depending on when the sensor 20 is outputting the response value corresponding to the state where no puffing takes place and when the sensor 20 is outputting the response value corresponding to the state where puffing takes place. The "light emitting state" includes, for example, the color of light emitted by the light-emitting element 40, the number of light-emitting elements 40 emitting light, the light quantity of the light emitting element 40, the cycle of turning on and off the light-emitting element 40, and the flashing pattern of the light-emitting element 40. Furthermore, the control circuit 50, for example, supplies power from the power source 10 to the atomization unit 120 (heater 120R) when the sensor 20 is outputting the response value corresponding to the state where puffing takes place, and stops supplying power from the power source 10 to the atomization unit 120 (heater 120R) when the sensor 20 is outputting the response value corresponding to the state where no puffing takes place.

The atomization unit 120 is, as shown in FIG. 1, connected to the electrical unit 110 so that the non-mouthpiece side of the atomization unit 120 and the mouthpiece side of the electrical unit 110 overlap with each other.

The atomization unit 120 includes, as shown in FIG. 3, a second tubular body 120X, a third tubular body 120Y, a mouthpiece 120A, a connector 120C, an elastic member 120E, a support 120F, a conduit 120G, conductive wires 120H and 120I, a reservoir 120P, a wick 120Q, and a heater 120R.

For example, the second tubular body 120X has a diameter approximately equal to that of the first tubular body 110X shown in FIGS. 1 and 2.

The mouthpiece 120A is provided in an opening portion on the mouthpiece side of the second tubular body 120X. For example, the mouthpiece 120A is fitted into the opening portion on the mouthpiece side of the second tubular body 120X. The mouthpiece 120A is provided with a gas outlet port 120B that connects the internal space of the second tubular body 120X and the external space of the flavor inhaler 100. Aerosol containing the tobacco flavor component is discharged via this gas outlet port 120B from the flavor inhaler 100 during inhalation.

The connector 120C is provided in the opening portion on the non-mouthpiece side of the second tubular body 120X. The connector 120C is, for example, fitted into the opening portion on the non-mouthpiece side of the second tubular body 120X. The connector 120C plays a role of connecting the atomization unit 120 to the electrical unit 110. In addition, the connector 120C has conductivity, and plays a role as a part of a conductive path for feeding power to the heater 120R. The center portion of the connector 120C is provided with a through hole for connecting the external space of the atomization unit 120 and a later-described atomization chamber.

The elastic member 120E is fitted into the through hole of the connector 120C. The elastic member 120E has rubber elasticity, and is electrically insulative. The center portion of the elastic member 120E is provided with a through hole.

The support 120F is fitted into the through hole of the elastic member 120E. The support 120F is provided with a through hole. The support 120F supports the conduit 120G in the through hole.

The opening on the non-mouthpiece side of the conduit 120G is a gas inlet port 120D that connects the internal space on the mouthpiece side of the electrical unit 110 and a later-described atomization chamber. During inhalation, the conduit 120G guides the air supplied from the electrical unit 110 to the atomization chamber, which is the inner space of the third tubular body 120Y. In addition, the conduit 120G has conductivity, and plays a role as a part of a conductive path for feeding power to the heater 120R.

The third tubular body 120Y is provided in the second tubular body 120X, and between the mouthpiece 120A and the connector 120C. The third tubular body 120Y is, for example, held by the mouthpiece 120A and the connector 120C. The wall of the third tubular body 120Y is provided with a pair of through holes on the non-mouthpiece side thereof. The third tubular body 120Y is made of a heat-resistant material such as a ceramic containing alumina as a primary component, for example.

The third tubular body 120Y forms a double tube structure together with the second tubular body 120X. In addition, the third tubular body 120Y and the second tubular body 120X are spaced apart from each other, and form a space surrounded by the mouthpiece 120A and the connector 120C. Hereinafter, this space will be referred to as a "liquid container", while the space inside the third tubular body 120Y will be referred to as an "atomization chamber".

The reservoir 120P is contained in the liquid container, and holds a liquid aerosol source. For example, the reservoir 120P is a porous body made of a material such as a resin web. The aerosol source is a liquid for forming aerosol, and does not contribute to tobacco flavor. The aerosol source is a liquid such as water, glycerin or propylene glycol.

The wick 120Q is disposed such that the wick penetrates a pair of through holes provided in the third tubular body 120Y, such that both ends thereof are located in the liquid container, and such that the center portion thereof is located in the atomization chamber. Both ends of the wick 120Q are in contact with the reservoir 120P.

In the central portion of the wick 120Q, the length direction thereof is approximately perpendicular to the air flow direction in the atomization unit 120.

The wick 120Q is constituted by a flavor source including a tobacco material. The wick 120Q sucks up the aerosol source held by the reservoir 120P at both ends of the wick 120Q by utilizing capillary action, and guides the sucked aerosol source to the central portion.

The liquid container containing the reservoir 120P may not include an additional flavor source. That is, the liquid container may include only the aerosol source held in reservoir 120P.

Alternatively, the liquid container may include an additional flavor source. The additional flavor source is, for example, "a flavor source including a tobacco material", "a flavor source other than a tobacco material", or both of them.

If the liquid container includes "a flavor source including a tobacco material" as an additional flavor source, the flavor source including the tobacco material is preferably a flavor source including leaf tobacco, a flavor source including a molded body made from a raw material including leaf tobacco, or a flavor source including an extract of leaf tobacco, more preferably a flavor source including a molded body made from a raw material including leaf tobacco. As mentioned above, the "leaf tobacco" used herein refers to a tobacco material which is ready to be incorporated into a flavor inhaler such as a heating-type flavor inhaler through various processing including a drying process of harvested tobacco leaves in a farm house, thereafter an aging process for 1 to several years in a leaf processing plant, and thereafter blending and cutting processes in a manufacturing plant. The "molded body made from a raw material including leaf tobacco" can be prepared by forming a raw material including leaf tobacco according to the publicly-known process. The molded body may have any shape such as a bead-like molded body, a sheet-like molded body, one obtained by cutting a sheet-like molded body into fibers, one obtained by cutting a sheet-like molded body into small pieces, and the like, as is publicly known in the art. Also, the "extract of leaf tobacco" can be prepared by extracting a leaf tobacco component from leaf tobacco according to techniques publicly known in the art.

If the liquid container includes a "flavor source other than a tobacco material" as an additional flavor source, the flavor source other than the tobacco material is preferably a flavor component contained in a liquid of an existing liquid-type electronic cigarette, for example, a flavorant such as menthol.

The heater 120R is, for example, a resistive heating element (for example, a heating wire) wound around the wick 120Q at a predetermined pitch. Power is supplied to the heater 120R from the power source 10 in response to the sensing of the user's inhalation action by the sensor 20 under the control of the control circuit 50. The heater 120R generates heat as power is supplied, and atomizes the aerosol source sucked up by the wick 120Q.

The conductive wire 120H electrically connects one end of the heater 120R and the conduit 120G. Furthermore, the conductive wire 120I electrically connects the other end of the heater 120R and the connector 120C. Power is supplied to the conductive wires 120H and 120I from the electrical unit 110 via the conduit 120G and the connector 120C, respectively.

[Wick]

As described above, in the present embodiment, the wick 120Q has the feature that it is constituted by the flavor source including a tobacco material. Hereinafter, details of the wick 120Q will be described.

The tobacco material included in the wick 120Q may be leaf tobacco, or a molded body made from a raw material including leaf tobacco (hereinafter, also referred to as "tobacco molded body"). As mentioned above, the "leaf tobacco" used herein refers to a tobacco material which is ready to be incorporated into a flavor inhaler such as a heating-type flavor inhaler through various processing including a drying process of harvested tobacco leaves in a farm house, thereafter an aging process for 1 to several years in a leaf processing plant, and thereafter blending and cutting processes in a manufacturing plant.

When the tobacco material included in the wick 120Q is leaf tobacco, the flavor source including leaf tobacco is contained in, for example, a heat-resistant mesh bag or a heat-resistant mesh container. The mesh bag or the mesh container has a mesh size which allows passage of the aerosol source and air but does not allow passage of the leaf tobacco (that is, the leaf tobacco does not fall out of the bag or container).

When the tobacco material included in the wick 120Q is a tobacco molded body, the tobacco molded body can be prepared by forming raw materials including leaf tobacco according to a publicly-known process. For example, the tobacco molded body can be prepared by mixing raw materials including leaf tobacco and rolling the resulting mixture into a sheet, followed by drying. Alternatively, the tobacco molded body can be prepared by subjecting leaf tobacco to extraction with water to obtain a tobacco extract and a residue, beating the resulting residue, subjecting it to a paper-making process to obtain a sheet made by the paper-making process, and adding a concentrate of the tobacco extract thereto. It is desirable that the amount of the concentrate to be added does not exceed the amount of the concentrate obtained from the tobacco extract obtained concurrently with the residue.

The tobacco molded body may be a molded body made from leaf tobacco, or a molded body made from a mixture of leaf tobacco and the absorbent material. When a molded body made from a mixture of the leaf tobacco and the absorbent material is used as the tobacco material included in the wick 120Q, the mass ratio of the leaf tobacco to the absorbent material can be set to 1:3 to 3:1. If a molded body made from a mixture of the leaf tobacco and the absorbent material is used as the tobacco material included in the wick 120Q, the absorbent material assists in the liquid-absorbing ability of the wick 120Q, and the user can thereby feel sufficient tobacco flavor. As the absorbent material, any fiber excellent in water absorbability can be used, an example of which includes cotton.

The tobacco molded body included in the wick 120Q may be a sheet-like molded body. The sheet-like molded body has a thickness of, for example, 0.5 to 2 mm. The tobacco molded body included in the wick 120Q may be one obtained by cutting a sheet-like molded body into a size suitable for the wick 120Q and laminating them (that is, a laminate of sheet-like molded bodies). Alternatively, the tobacco molded body included in the wick 120Q may be a sheet-like molded body wound in a spiral shape, or a sheet-like molded body folded in a bellows shape. Alternatively, the tobacco molded body included in the wick 120Q may be one obtained by cutting a sheet-like molded body into a fibrous shape and bundling the obtained fibrous molded bodies (that is, a bundle of fibrous molded bodies). The fibrous molded bodies can be obtained by cutting a sheet-like molded body to, for example, a length of 3 to 5 cm and a width of 0.5 to 2 mm. As described above, when a bundle of fibrous molded bodies is used as the wick 120Q, the wick 120Q in this form exhibits an excellent liquid-absorbing ability because of its large specific surface area, and this allows a user to feel sufficient tobacco flavor.

The wick 120Q may include, in addition to the tobacco material, a substance capable of assisting in the liquid-absorbing ability of the wick 120Q. For example, the wick 120Q may include salt in addition to the tobacco material. For the salt, it is possible to use potassium carbonate, sodium hydroxide, calcium oxide, and the like. If the tobacco material contained in wick 120Q is leaf tobacco, it is possible to add a salt-containing liquid to the leaf tobacco. Alternatively, if the tobacco material included in the wick 120Q is a tobacco molded body, it is possible to add salt during preparation of the tobacco molded body. The salt can be added so that an aqueous solution obtained by adding 10 times the weight of water to a salt-added tobacco material is preferably alkaline or neutral, more preferably at a pH of 6 to 9. For example, the salt can be added in an amount of 5% to 10% by mass, based on the tobacco material. When added to the tobacco material, the above-described salt can assist in the liquid-absorbing ability of the wick 120Q, and can suppress proliferation of microorganisms.

The amount of the "flavor source including a tobacco material" constituting the wick 120Q can be appropriately determined according to the form of the flavor inhaler, and it can be set to 5 to 300 mg, for example.

[Advantageous Effects]

As described above, in the embodiment of the present invention, the wick 120Q is constituted by a flavor source including the tobacco material. Therefore, when the aerosol source permeates into the wick 120Q, the aerosol source functions as an extraction solvent, and the tobacco flavor component is extracted from the tobacco material included in the wick 120Q. At the time of inhalation, the aerosol source including the tobacco flavor component is vaporized by the heat generated by heater 120R, and then cooled and atomized (aerosolized). In this manner, the user can enjoy the tobacco flavor.

In addition, according to the embodiment of the present invention, the flavor source including the tobacco material is used as the wick 120Q, and it is thus possible to taste sufficient tobacco flavor without adding an additional flavor source to the liquid container. In the embodiment of the present invention, when an additional flavor source is not added to the liquid container, there are advantages such as absence of contamination in the liquid container due to the attachment of the additional flavor source and no clogging in the wick 120Q due to the attachment of the additional flavor source.

Alternatively, as described above, according to the embodiment of the present invention, an additional flavor source can be included in the liquid container. Specifically, in the liquid container, any additional flavor source can be included such as "a flavor source including a tobacco material" or "a flavor source other than a tobacco material". In this case, the combination of the tobacco material included in the wick 120Q and the additional flavor source included in the liquid container can provide variations in the tobacco flavor that the user enjoys.

[Examples]

[1] Manufacture of Wick

Example 1: Silica fiber

In Example 1, a silica fiber (Braided Silica Wick for e-Cig Atomizer (D = 3.0 mm)) was used as a wick.

Example 2: Tobacco molded body made from leaf tobacco

In Example 2, the tobacco molded body was prepared as described below and used as a wick.
The leaf tobacco (BRBLA (Brazilian Burley)) was subjected to extraction with a ten-fold volume of warm water at 60°C, and separated into a tobacco extract and a residue. The obtained residue was beaten with a refiner. The beaten residue was subjected to a paper-making process by paper machine and dried to obtain a sheet made by the paper-making process. Meanwhile, the obtained tobacco extract was concentrated to obtain a concentrate. The concentrate was added to the obtained sheet to obtain a sheet-like tobacco molded body (thickness: approximately 1 mm). In this example, as for the concentrate, the whole amount of the concentrate obtained from the tobacco extract obtained concurrently with the residue was added.
The resulting sheet-like tobacco molded body was cut to a width of approximately 0.5 to 2 mm and a length of approximately 3 to 5 cm. As a result, a fibrous tobacco molded body was obtained.

Example 3: Tobacco molded body with potassium carbonate added

In Example 3, a fibrous tobacco molded body was obtained in the same manner as in Example 2, except that potassium carbonate was added to the beaten residue in an amount of 5% by mass based on the leaf tobacco followed by reaction for 2 hours.

Example 4: Tobacco molded body with potassium carbonate added

In Example 4, a fibrous tobacco molded body was obtained in the same manner as in Example 2, except that potassium carbonate was added to the beaten residue in an amount of 10% by mass based on the leaf tobacco followed by reaction for 2 hours.

Example 5: Tobacco molded body made from mixture of leaf tobacco and cotton

In Example 5, a fibrous tobacco molded body was obtained in the same manner as in Example 2, except that the beaten residue and cotton ("Clé de Peau Beauté Le Coton" manufactured by Shiseido Company, Limited) were mixed at a mass ratio of 3:1 and the resulting mixture was subjected to a paper-making process by paper machine.

Example 6: Tobacco molded body made from mixture of leaf tobacco and cotton

In Example 6, a fibrous tobacco molded body was obtained in the same manner as in Example 2, except that the beaten residue and the cotton were mixed at a mass ratio of 1:1 and the resulting mixture was subjected to a paper-making process by paper machine.

Example 7: Tobacco molded body made from mixture of leaf tobacco and cotton

In Example 7, a fibrous tobacco molded body was obtained in the same manner as in Example 2, except that the beaten residue and the cotton were mixed at a mass ratio of 1:3 and the resulting mixture was subjected to a paper-making process by paper machine.

Example 8: Tobacco molded body with potassium carbonate added and made from mixture of leaf tobacco and cotton

In Example 8, a fibrous tobacco molded body was obtained in the same manner as in Example 2, except that potassium carbonate was added to the beaten residue in an amount of 10% by mass based on the leaf tobacco followed by reaction for 2 hours, and that the reacted residue and the cotton were mixed at a mass ratio of 1:1 followed by subjecting the resulting mixture to a paper-making process by paper machine.

[2] Evaluation of liquid-absorbing ability of wick

The liquid-absorbing ability of the wick of Examples 1 to 8 was evaluated as follows.

35 mg of the wick was immersed in approximately 100 mL of an aerosol forming liquid (water, glycerin, or propylene glycol). After 30 seconds, the wick was taken out from the aerosol forming liquid and lightly drained, and the weight of the wick was measured. The liquid absorption amount per 1 g of the wick was calculated by the following equation:

$$\texttt{Liquid absorption amount (g) per 1 g of wick =}$$
$$\texttt{\{(Weight of wick after immersion (mg)) - (Weight of wick}$$
$$\texttt{before immersion (mg))\} / 35}$$

Table 1 shows the liquid absorption amount (g) per 1 g of the wick when each aerosol forming liquid was used.

[Table 1]

| Example | Liquid absorption amount (g) per 1 g of wick when each aerosol forming liquid is used | | |
| --- | --- | --- | --- |
| | Propylene glycol | Glycerin | Water |
| 1 | 1.1 | 1.9 | 1.1 |
| 2 | 1.4 | 1.1 | 1.2 |
| 3 | 2.8 | 1.8 | 2.6 |
| 4 | 3.6 | 2.2 | 4.7 |
| 5 | 8.2 | 8.1 | 8.0 |
| 6 | 7.9 | 10.4 | 8.3 |
| 7 | 9.4 | 11.7 | 10.5 |
| 8 | 9.4 | 12.3 | 10.2 |

From the results of Example 1 and Example 2, it is understood that the tobacco molded body exhibits the liquid-absorbing ability equivalent to that of the conventional wick (silica fiber). From the results of Examples 2 to 4, it is understood that when potassium carbonate is added to the tobacco molded body, potassium carbonate can assist in the liquid-absorbing ability. From the results of Example 2 and Examples 5 to 7, it is understood that when cotton is incorporated into the tobacco molded body, the cotton can assist in the liquid-absorbing ability. From the result of Example 8, it is understood that when potassium carbonate is added to the tobacco molded body and cotton is incorporated into the tobacco molded body, potassium carbonate and cotton can each assist in the liquid-absorbing ability.

[3] Manufacture of heating-type flavor inhaler

Using the wick of Examples 1 to 8 (approximately 60 mg), the heating-type flavor inhaler shown in FIGS. 1 to 3 was manufactured. An equal mixture of glycerin and propylene glycol was used as an aerosol source.

[4] Sensory evaluation

The sensory evaluation was performed on the tobacco-like flavor by inhaling the heating-type flavor inhaler.

When the wick of Example 1 was used, the tobacco-like flavor was not tasted, but the dust-like flavor was tasted. When the wick of Example 2 was used, the sufficient tobacco-like flavor was tasted. When the wicks of Examples 3 to 8 were used, the sufficient tobacco-like flavor was tasted like the wick of Example 2. The reason why the sufficient tobacco-like flavor was tasted with the wicks in Examples 5 to 8 is considered to be that their wicks have superior liquid-absorbing ability because of the cotton mixed although the amount of the leaf tobacco is less in comparison to the wick of Example 2.

## Claims

1. An atomization unit (120) for use in a heating-type flavor inhaler (100) provided with a power source (10), and atomizing a liquid aerosol source by power supplied from the power source (10), the atomization unit (120) comprising:

   an atomization chamber (120Y) having a gas inlet port and a gas outlet port, and containing a flavor source (120Q) including a tobacco material;
   a liquid container (120Y, 120X) containing a liquid aerosol source, and supplying the aerosol source to the flavor source(120Q); and
   a heater (120R) heating the flavor source(120Q) supplied with the aerosol source to atomize the aerosol source and to release a flavor component from the flavor source (120Q) .

2. The atomization unit (120) according to claim 1, wherein the tobacco material is a molded body made from a raw material including leaf tobacco.

3. The atomization unit (120) according to claim 2, wherein the molded body is a molded body made from leaf tobacco.

4. The atomization unit (120) according to claim 2, wherein the molded body is a molded body made from a mixture of leaf tobacco and an absorbent material.

5. The atomization unit (120) according to claim 4, wherein the absorbent material is cotton.

6. The atomization unit (120) according to any one of claims 2 to 5, wherein the molded body is a bundle of fibrous molded bodies.

7. The atomization unit (120) according to any one of claims 1 to 6, wherein the liquid container (120Y, 120X) supplies the aerosol source to the flavor source(120Q) by immersing a part of the flavor source(120Q) in the aerosol source.

8. The atomization unit (120) according to any one of claims 1 to 7, wherein the heater (120R) is a resistive heating element wound around the flavor source(120Q).

9. The atomization unit (120) according to any one of claims 1 to 8, wherein the flavor source(120Q) contains salt.

10. The atomization unit (120) according to any one of claims 1 to 9, wherein the liquid container (120Y, 120X) includes only the aerosol source.

11. The atomization unit (120) according to any one of claims 1 to 9, wherein the liquid container (120Y, 120X) further includes an additional flavor source(120Q).

12. The atomization unit (120) according to claim 11, wherein the additional flavor source(120Q) is a flavor source(120Q) including a tobacco material.

13. The atomization unit (120) according to claim 12, wherein the flavor source(120Q) including the tobacco material is a flavor source(120Q) including a molded body made from a raw material including leaf tobacco.

14. The atomization unit (120) inhaler according to any one of claims 1 to 13, wherein the liquid container (120Y, 120X) further includes a reservoir that holds the aerosol source.

15. A heating-type flavor inhaler (100) comprising the atomization unit (120) according to any one of claims 1 to 14 and a power source (10) that supplies power to the heater (120R).

## Patentansprüche

1. Zerstäubungseinheit (120) zur Verwendung in einem Aroma-Inhalator (100) vom Heizungstyp, der mit einer Energiequelle (10) bereitgestellt ist, und zum Zerstäuben einer Quelle flüssigen Aerosols durch von der Energiequelle (10) zugeführte Energie, wobei die Zerstäubungseinheit (120) umfasst:

eine Zerstäubungskammer (120Y), die eine Gaseinlassöffnung und eine Gasauslassöffnung aufweist und eine Aromaquelle (120Q) einschließlich eines Tabakmaterials enthält;

einen Flüssigkeitsbehälter (120Y, 120X), der eine Quelle flüssigen Aerosols enthält und die Aerosolquelle zu der Aromaquelle (120Q) zuführt und

ein Heizaggregat (120R), das die der Aerosolquelle zugeführte Aromaquelle (120Q) erhitzt, um die Aerosolquelle zu zerstäuben und um eine Aromakomponente aus der Aromaquelle (120Q) freizusetzen.

2. Zerstäubungseinheit (120) nach Anspruch 1, wobei das Tabakmaterial ein Formkörper ist, der aus einem Rohmaterial einschließlich Tabakblättern hergestellt ist.

3. Zerstäubungseinheit (120) nach Anspruch 2, wobei der Formkörper ein Formkörper ist, der aus Tabakblättern hergestellt ist.

4. Zerstäubungseinheit (120) nach Anspruch 2, wobei der Formkörper ein Formkörper ist, der aus einer Mischung aus Tabakblättern und einem absorbierenden Material hergestellt ist.

5. Zerstäubungseinheit (120) nach Anspruch 4, wobei das absorbierende Material Baumwolle ist.

6. Zerstäubungseinheit (120) nach einem der Ansprüche 2 bis 5, wobei der Formkörper ein Bündel von faserigen Formkörpern ist.

7. Zerstäubungseinheit (120) nach einem der Ansprüche 1 bis 6, wobei der Flüssigkeitsbehälter (120Y, 120X) die Aerosolquelle zu der Aromaquelle (120Q) zuführt, indem ein Teil der Aromaquelle (120Q) in die Aerosolquelle getaucht wird.

8. Zerstäubungseinheit (120) nach einem der Ansprüche 1 bis 7, wobei das Heizaggregat (120R) ein um die Aromaquelle (120Q) gewickeltes Widerstandsheizelement ist.

9. Zerstäubungseinheit (120) nach einem der Ansprüche 1 bis 8, wobei die Aromaquelle (120Q) Salz enthält.

10. Zerstäubungseinheit (120) nach einem der Ansprüche 1 bis 9, wobei der Flüssigkeitsbehälter (120Y, 120X) nur die Aerosolquelle einschließt.

11. Zerstäubungseinheit (120) nach einem der Ansprüche 1 bis 9, wobei der Flüssigkeitsbehälter (120Y, 120X) weiter eine zusätzliche Aromaquelle (120Q) einschließt.

12. Zerstäubungseinheit (120) nach Anspruch 11, wobei die zusätzliche Aromaquelle (120Q) eine Aromaquelle (120Q) ist, die ein Tabakmaterial einschließt.

13. Zerstäubungseinheit (120) nach Anspruch 12, wobei die das Tabakmaterial einschließende Aromaquelle (120Q) eine Aromaquelle (120Q) ist, die einen Formkörper einschließt, der aus einem Rohmaterial einschließlich Tabakblättern hergestellt ist.

14. Inhalator mit Zerstäubungseinheit (120) nach einem der Ansprüche 1 bis 13, wobei der Flüssigkeitsbehälter (120Y, 120X) weiter ein Reservoir einschließt, das die Aerosolquelle hält.

15. Aromainhalator (100) vom Heizungstyp, umfassend die Zerstäubungseinheit (120) nach einem der Ansprüche 1 bis 14 und eine Stromquelle (10), die Strom zu dem Heizaggregat (120R) zuführt.

**Revendications**

1. Unité d'atomisation (120) destinée à être utilisée dans un inhalateur d'arôme de type chauffant (100) pourvue d'une source d'énergie (10) et atomisant une source d'arôme liquide par l'énergie fournie depuis la source d'énergie (10), l'unité d'atomisation (120) comprenant :

une chambre d'atomisation (120Y) présentant un orifice d'entrée de gaz et un orifice de sortie de gaz, et contenant une source d'arôme (120Y) incluant un matériau de tabac ;

un contenant de liquide (120Y, 120X) contenant une source d'aérosol liquide et fournissant la source d'aérosol à la source d'arôme (120Q) ; et

un dispositif de chauffage (120R) chauffant la source d'arôme (120Q) fournie avec la source d'aérosol pour atomiser la source d'aérosol et pour libérer un composant d'arôme à partir de la source d'arôme (120Q).

2. Unité d'atomisation (120) selon la revendication 1, dans laquelle le matériau de tabac est un corps moulé réalisé à partir d'une matière première incluant du tabac en feuilles.

3. Unité d'atomisation (120) selon la revendication 2, dans laquelle le corps moulé est un corps moulé réalisé à partir du tabac en feuilles.

4. Unité d'atomisation (120) selon la revendication 2, dans laquelle le corps moulé est un corps moulé réalisé à partir d'un mélange de tabac en feuilles et d'un matériau absorbant.

5. Unité d'atomisation (120) selon la revendication 4, dans laquelle le matériau absorbant est du coton.

6. Unité d'atomisation (120) selon l'une quelconque des revendications 2 à 5, dans laquelle le corps moulé est un faisceau de corps moulés fibreux.

7. Unité d'atomisation (120) selon l'une quelconque des revendications 1 à 6, dans laquelle le contenant de liquide (120Y, 120X) fournit la source d'aérosol à la source d'arôme (120Q) en immergeant une partie de la source d'arôme (120Q) dans la source d'aérosol.

8. Unité d'atomisation (120) selon l'une quelconque des revendications 1 à 7, dans laquelle le dispositif de chauffage (120R) est un élément chauffant résistif enroulé autour de la source d'arôme (120Q).

9. Unité d'atomisation (120) selon l'une quelconque des revendications 1 à 8, dans laquelle la source d'arôme (120Q) contient du sel.

10. Unité d'atomisation (120) selon l'une quelconque des revendications 1 à 9, dans laquelle le contenant de liquide (120Y, 120X) inclut seulement la source d'aérosol.

11. Unité d'atomisation (120) selon l'une quelconque des revendications 1 à 9, dans laquelle le contenant de liquide (120Y, 120X) inclut en outre une source d'arôme supplémentaire (120Q).

12. Unité d'atomisation (120) selon la revendication 11, dans laquelle la source d'arôme supplémentaire (120Q) est une source d'arôme (120Q) incluant un matériau de tabac.

13. Unité d'atomisation (120) selon la revendication 12, dans laquelle la source d'arôme (120Q) incluant le matériau de tabac est une source d'arôme (120Q) incluant un corps moulé réalisé à partir d'une matière première incluant du tabac en feuilles.

14. Inhalateur d'unité d'atomisation (120) selon l'une quelconque des revendications 1 à 13, dans lequel le contenant de liquide (120Y, 120X) inclut en outre un réservoir qui contient la source d'aérosol.

15. Inhalateur d'arôme de type chauffant (100) comprenant l'unité d'atomisation (120) selon l'une quelconque des revendications 1 à 14 et une source d'énergie (10) qui fournit de l'énergie au dispositif de chauffage (120R).

F I G. 1

F I G. 2

EP 3 545 777 B1

FIG. 3

**EP 3 545 777 B1**

**REFERENCES CITED IN THE DESCRIPTION**

**Patent documents cited in the description**

- WO 2013116558 A1
- US 2014157583 A1